# EUROPEAN PATENT APPLICATION

(11) **EP 2 995 254 A1**
(43) Date of publication of application: **16.03.2016**
(21) Application number: 14184514.9
(22) Date of filing: 12.09.2014
(51) Int. Cl.: A61B 6/00, A61B 6/02

(54) **Imaging system**

(71) Applicant: Koninklijke Philips N.V., 5656 AE Eindhoven (NL)
(72) Inventor: KOEHLER, Thomas, 5600 AE Eindhoven (NL); GRASS, Michael, 5600 AE Eindhoven (NL); PROKSA, Roland, 5600 AE Eindhoven (NL)
(74) Representative: Gravendeel, Cornelis

(57) **Abstract**

The present invention relates to a stereo tube radiation imaging system in which radiation emitted from each radiation source covers a different area of the detector surface. Furthermore, the present invention relates to a stereo tube imaging method wherein both radiation sources are operated independently and each cover part of the detector surface area. This is advantageous in that it may reduce radiation dose compared to known stereo tube imaging and introduces new possibilities for stereo tube imaging, such as improved object tracking within a body.

## Description

### FIELD OF THE INVENTION

The present invention generally relates to an imaging system comprising a detection unit comprising a detector surface and a radiation unit comprising a first radiation emission region configured to emit radiation towards a first area of the detector surface area, and a second radiation emission region configured to emit radiation towards a second area of the detector surface area. The present invention is further directed towards a method to scan an object of interest with said imaging system.

### BACKGROUND OF THE INVENTION

In radiation imaging systems, such as X-ray imaging system or computed tomography, radiation is usually emitted in the form of a beam, for instance a cone beam that may be collimated. A known variation thereof is a so-called stereo tube radiation imaging system comprising an x-ray radiation unit having two focal spots, wherein a region of interest is illuminated by the radiation emanating from the two focal spots. The radiation of the two focal spots after having traversed the region of interest is detected by a detection unit. Since the region of interest is imaged simultaneously by two, off-set radiation beams, it is possible to generate a stereoscopic (3D) image of the region of interest from detected radiation data.

Stereo tube radiation imaging devices have not yet been widely implemented, because the advantages of stereo imaging are often outweighed by disadvantages, such as increased radiation dose and technical complexities.

### SUMMARY OF THE INVENTION

It is an object of the present invention to leverage the balance between the advantages and disadvantages of stereo tube imaging, e.g. by decreasing the radiation dose or by adding unique features only obtainable by stereo tube imaging, which could improve the acceptance of stereo tube imaging.

Embodiments according to the present invention are directed to an imaging system according to claims 1-7.

Another embodiment of the present invention is directed towards a method to scan an object of interest with an imaging system according to claims 8-15.

Still further aspects and embodiments of the present invention will be appreciated by those of ordinary skill in the art upon reading and understanding the following detailed description. Numerous additional advantages and benefits will become apparent to those of ordinary skill in the art upon reading the following detailed description of preferred embodiments.

### BRIEF DESCRIPTION OF THE DRAWINGS

The present invention is illustrated by drawings of which
Figure 1 shows an exemplary stereo tube computed tomography imaging system;
Figures 2a-d show schematic embodiments of operating a radiation unit with two or more sources and a detection unit, of which fig. 2a depicts known operation;
Figures 3a-d show other schematic embodiments of operating a radiation unit with two sources and a detection unit;
Figures 4a-d show a schematic sequence of imaging an object travelling through a body according to the present invention, wherein the object has a lower speed for part of the path through the body due to a narrowing;
Figures 5a-d show a schematic sequence of imaging an object travelling through a body according to the present invention, wherein the object has a higher speed for part of the path through the body due to a widening;
Figures 6a-d show a schematic sequence of imaging an object travelling through a body according to an embodiment of the present invention, wherein the radiation unit has three radiation sources.
Figure 7 shows a flowchart of a method to scan an object of interest with an imaging system according to the present invention;
Figure 8 shows a flowchart of a method to track an object of interest travelling through a body with an imaging system according to the present invention.

The invention may take form in various components and arrangements of components, and in various process operations and arrangements of process operations. The drawings are only for the purpose of illustrating preferred embodiments and are not to be construed as limiting the invention. To better visualize certain features may be omitted or dimensions may be not be according to scale.

### DETAILED DESRIPTION OF EMBODIMENTS

The present invention is explained using a stereo tube computed tomography (CT) system as an example. The invention is suitable for use in other radiation-based imaging systems as well, including interventional and diagnostic X-ray imaging systems or combinations including these or CT.

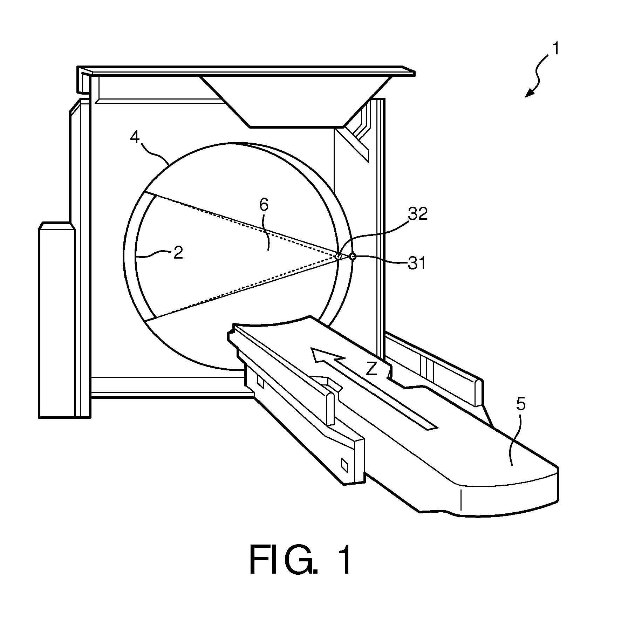

Figure 1 shows a schematic depiction of a known, stereo tube computed tomography device 1. An x-ray radiation detector 2 and an x-ray unit 31, 32 are mounted on a rotatable gantry 4. A body to be scanned, such as a patient, is positioned on movable bench 5, which during scanning moves in the z-direction through examination region 6, while gantry 4 rotates around the examination area and x-ray radiation is emitted from two separate x-ray emission regions 31, 32 that are spatially offset on the gantry 4 in the z-direction. This may be implemented by using two radiation sources each having its own focal spot or one source with two focal spots or by using an external filter system or collimator system. The x-ray radiation which passes through the subject is detected by x-ray detector 2, in which the detected x-ray radiation is converted to electronic information that is further processed in further processing equipment (not shown) to visual information which is displayed to a user, such as a physician. Detector 2 may convert x-ray radiation into electronic information using various principles, such as scintillation, in which x-ray radiation is converted into radiation at another wavelength. An alternative conversion method is direct conversion, in which x-rays are directly converted into electrons in a direct conversion material (such as Cadmium Zinc Telluride or Cadmium Telluride).

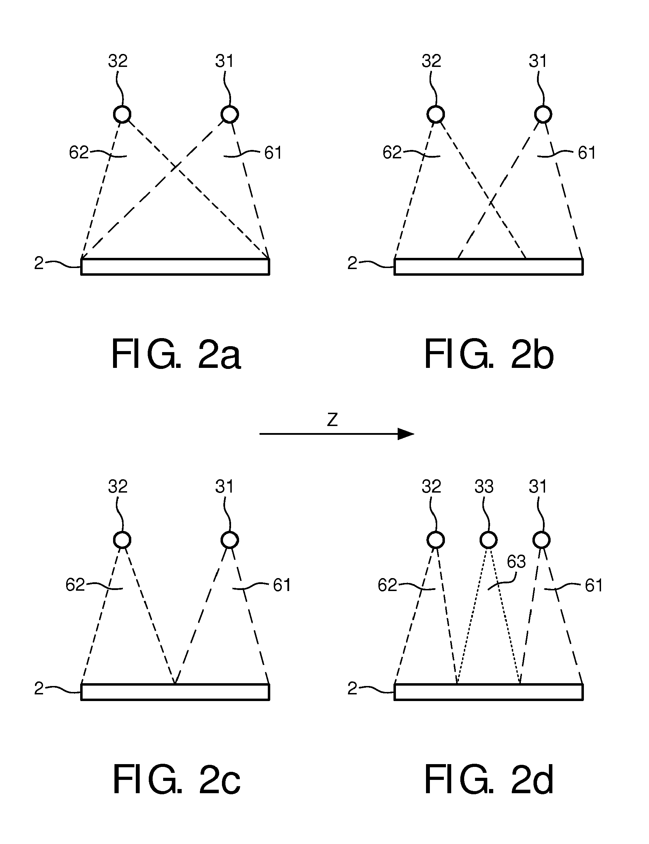

Figure 2a illustrates known operation of a stereo tube imaging system, whereby radiation from both radiation emission regions 31, 32 is irradiated towards the detector 2, both substantially covering the full detector area, where the emission regions may operate in an alternating fashion. The thusly generated detection data may be reconstructed to form a three dimensional image that might provide additional insight into the scanned object (compared to a two dimensional image). A further advantage of such known stereo-tube imaging is that the system, at a given physical detector size provides a larger coverage (i.e., scanned volume per rotation) than a single source scanner. On the other hand, the effective cone-angle is increased by this and therefore, the reconstructed images may suffer more strongly from cone-beam artifacts than a single source scanner.

Figure 2b depicts an embodiment of the imaging system of the present invention that retains the advantages of stereo tube radiation imaging, but at a significantly lowered cone-angle, resulting in less cone-beam artifacts and thus being a preferred acquisition mode if scan time or coverage is less important than image quality (cone-beam artifacts).. In this embodiment radiation beams 61, 62 from radiation emission regions 31, 32 each illuminate only part of the surface of detector 2, thereby reducing the maximum cone angle of the rays from each emission region. Another potential problem with known stereo-tube imaging is that afterglow does no longer lead to just angular blurring, but it may cause artifacts since effectively, a small fraction from the other focal spot might always be visible in the projection data. This might become a problem in particular for cases where demands on image quality are extremely high (e.g. head imaging). This problem is also overcome by having different sections of the detector surface illuminated from the two emission regions 31, 32. The smaller the overlap, the smaller the chance of artifacts due to afterglow.

In figure 2b both radiation beams 61, 62 are symmetric and cover a detector surface area substantially equal of size. However, radiation from each emission region 31, 32 may be differently, or even variably collimated to obtain radiation beams 61, 62 that they cover different sized areas of the detector surface. This may be advantageous to direct most radiation to a desired detector surface area, while radiation dose received at other areas is lower.

The embodiment shown in figure 2c is a particularly advantageous embodiment of the imaging system of the present invention, wherein substantially adjacent parts of the detector are illuminated from each of the radiation emission regions 31, 32. In this acquisition mode, both emission regions 31, 32 can operate simultaneously, which improves the angular sampling of the data, leading to less sampling artifacts and higher spatial resolution in the reconstructed images. At the same time, the coverage and thus the scan speed to two times better than a single source system with the same cone-angle as the cone angle of each of the two emission regions while providing the same image quality. The two radiation beams 61, 62 might be collimated even stronger, leading to a smaller cone-angle, but at the cost of increased scan time. In light of this embodiment the term substantially adjacent should be considered to mean that both radiation beams 61, 62 overlap not more than 10%, more preferably not more than 5% and most preferably no overlap (e.g. only touch at the edges), of the total detector surface area.

The imaging system of the present invention is not limited to having two radiation imaging regions 61, 62 As shown in figure 2d, a third radiation emission region 33 could be easily added, whereby each of the radiation beams 61, 62, 63 partly, preferably substantially adjacent) illuminate the detector surface. This may be advantageous to generate more options, some of which will be described further on in this document.

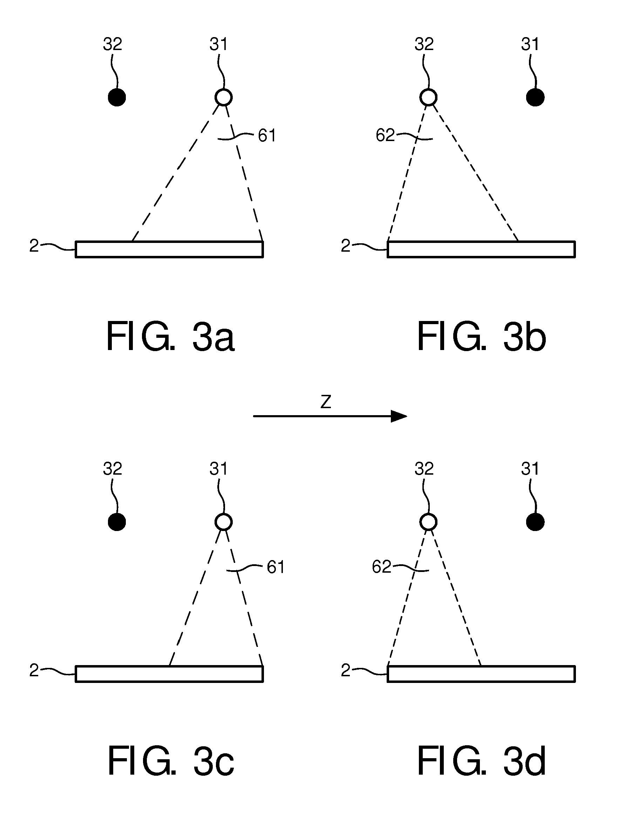

By illuminating only part of the detector surface from a radiation emission region 61, 62, further advantageous embodiments are available when the emission regions 61, 62 are operated independently, i.e. when not always radiation is emitted from them at the same time. Parts of the body may be scanned with only a single emission region switched on (as seen in figures 3a-d), for instance regions around the region of interest that are only necessary to provide context or do not need as detailed imaging as the region of interest (e.g. determining the start of the region of interest on approach). For regions of the body to be scanned that are of particular interest the second emission region may be switched on as well (as shown in figures 2b or 2c) to generate more image data for that region, allowing for a more refined image reconstruction. When the region of interest has moved out of the examining region 6, one of the beams 61, 62 may be switched off again. Obviously, radiation dose received per surface area is reduced when the overlap of the beams 61, 62 is smaller (such as in figures 3c-d compared to 3a-b), but also image quality will deteriorate with smaller or no overlap.

A particularly interesting option that arises with an imaging system of the present invention that is further capable of operating radiation emitted from each emission region separately, as described previously, is an improved capability of tracking objects travelling through a body, wherein said body itself is translated through the examination region 6 of the imaging system 1. This invention will be further explained using the example of tracking of a contrast agent bolus through a stenosed blood vessel. The invention is not limited to this example or even to medical imaging, it can easily be adapted to track any object travelling through any body, wherein a travelling speed of the object is locally changed, provided that the imaging system can detect the object inside the body.

Contrast agent bolus tracking is used in medical imaging to visualize blood flow through vessels 101 in a patient's body 100, and by this it provides means to quantify a functional impact of a stenosis. After injection of a contrast agent bolus 103 into a patient's blood vessel 101, it is followed by the imaging system from the moment it arrives at a predetermined intensity level in the examination region 6 of the imaging system 1. Imaging data is acquired at a rate corresponding to the rate of the bolus 103 moving through the blood vessel 101. The examination region normally does not change position with respect to the moving direction of the region of interest, although the source may circle the examination region 6, which means that, when the region of interest is translated at a constant speed through the examination region 6, the acquisition occurs at regular intervals, independent of region of interest characteristics.

Tracking the bolus 103, or in other words: ensuring that the bolus 103 remains in the examining region 6, is often complicated since the speed of the bolus 103 may not be constant though the blood vessel 101. It may be influenced by a local narrowing 102 of the blood vessel, e.g. due to a stenosis. The contrast agent bolus 103 usually speeds up when the arterial narrowing 102 is encountered. The bolus 103 may actually be too fast for an adequate visualization and the imaging system may 'outrun' the examining region. It may be necessary to influence the translation speed of the body 100 containing the blood vessel 101 based on the arterial anatomy, which is technically quite difficult to realize, especially since it is not always known beforehand where the arterial narrowing 102 is exactly located.

It would be conceivable that acquisition intervals, translation speed and determining the position of the region of interest are all determined and/or adapted during scanning to avoid running out of the bolus. However, this requires a precise interplay between each of these within seconds or even faster, which would require severe computational effort and is also mechanically not straightforward, since this would involve a serious redesign and cost increase of the imaging system.

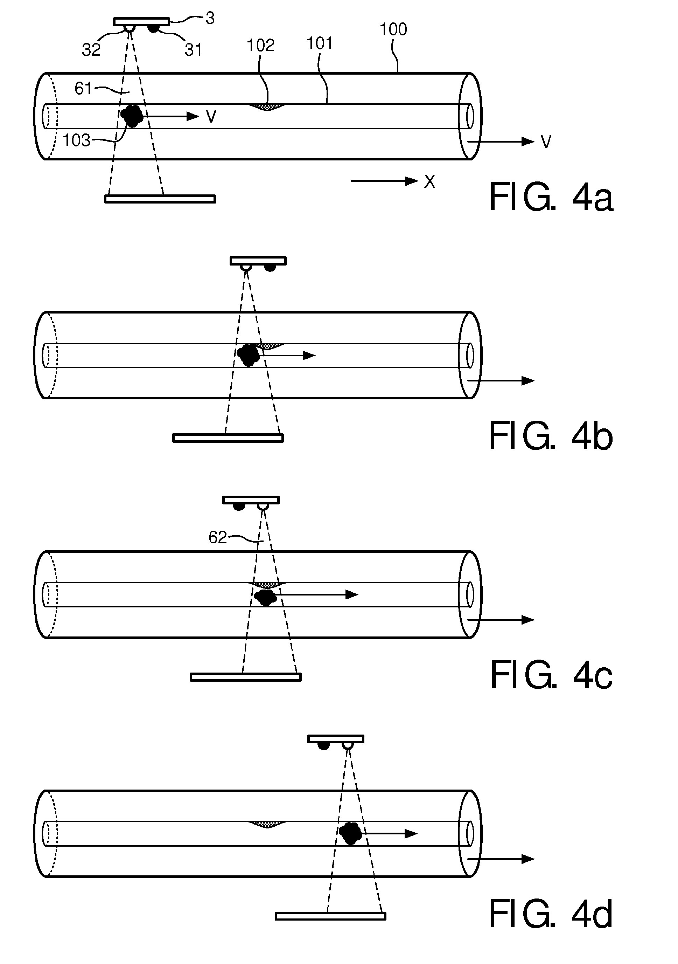

Figures 4a-d shows an embodiment of the present invention that improves bolus tracking without serious redesign of the imaging system, thereby adding an additional feature for stereo tube radiation imagers that could increase acceptance of such systems. In figures 4a-d a contrast agent bolus 103 is shown moving through a blood vessel 101, e.g. an artery or a vein, in a human or animal body 100. For clarity, the bolus 103 is schematically depicted as a deformable particle, which differs from the real-life situation, wherein the bolus 103 is more or less a fluid stretched out over a certain length with gradually increasing and decreasing opacity. The blood vessel 101 comprises a narrowing 102, in this example a stenosis, which has formed on the vessel wall. The contrast agent bolus travels in main direction x at object speed v. The body is translated through an examination region of a radiation imaging system comprising a detector 3 with a first emission region 31 and a second emission region 32 and detector 2. When the contrast agent bolus 103 arrives at the examination region 6 (or at least if contrast increases above a predetermined intensity level), the body 100 is moved through the examination region 6 in direction x and at speed v, such that the relative speed of the bolus 103 and the body 100 are substantially the same and the bolus 103 remains in the examination region 6 of the imaging system 1.

In figure 4a the bolus 103 is in a part of the blood vessel 101 before (proximal to) the stenosis 102. The bolus 103 is imaged by radiation beam 61, which is emitted solely from the first emission region 31. No radiation from emission region 32 is emitted through the examining region (e.g. by switching off emission region 32 or having a closed shutter in front of emission region 32). In this example the radiation beam 61 is collimated such that it only illuminates part, approximately covering the backward half, of the surface of detector 2 (corresponding to the embodiment shown in figure 3c). The radiation beam 61 may also be collimated such that is covers a smaller or larger (such as the complete) detector area of detector 2 (such as for instance corresponding with figure 3a).

In figure 3b the bolus 103 has arrived at the narrowing 102, causing object speed v to increase and the bolus 103 to outrun examining region 61. At this point, as is shown in figure 3c, the radiation unit 3 switches on emission region 32 to image the complete bolus 103 with radiation beam 62, which is also collimated such that it only illuminates part, now approximately covering the forward half, of the surface of detector 2 (corresponding to figure 3d). Emission region 31 may be switched off immediately or gradually (by reducing intensity), but may also be left switched on or operated at a lower intensity. Similarly, emission region 32 may be switched on immediately or gradually (by increasing intensity). As a consequence, not only the bolus 103 is always fully imaged, but also the area of and just around the narrowing is imaged longer and with more intensity, providing more data to form an image, while non-narrowed areas potentially receive less dose compared to a full illumination of the detector (at the same radiation intensity).

After (distal to) the narrowing 102 the speed v reverts to approximately the same as proximal to the stenosis, but, in the situation shown in figure 3d, the bolus 103 is still fully imaged by radiation beam 62. If the speed would decrease such that that the emission beam 62 would outrun the bolus 103, the radiation unit may switch on emission region 31 again to image the bolus (and optionally switch off emission region 62).

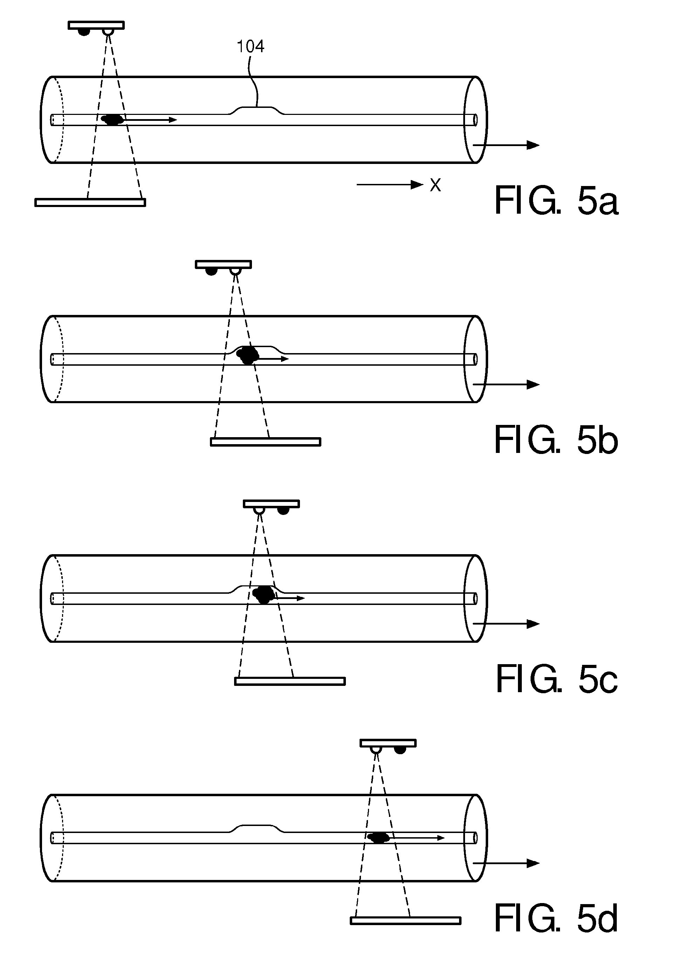

Figures 5a to 5d show a similar situation as in figures 4a to 4d, except that the blood vessel 101 now comprises a local widening 104, which causes the speed v to locally decrease and the examining beam 61 may outrun the bolus 103. This may be solved by switching on emission region 31 which is behind of emission region 32. This situation is further opposite, but analogous to that of the situation with a local narrowing as described previously and shown in figures 4a-4d.

Determining whether the bolus 103 is fully in the emission beam 61, 62 may be done inline, wherein a processing unit analyses detection data and determines whether the bolus 103 is still within the emission beam 61, 62 or not. This of course requires fast processing and immediate communication with drivers that are able to switch to emitting radiation from the other emission region.

Alternatively or additionally, the imaging device may comprise an object of interest location prediction unit that is capable of predicting a location of the object of interest relative to the examination region for at least part of a remaining length of the body through which the object of interest will travel. The switch between radiation emission regions 31, 32 can then be anticipated. The object of interest location prediction unit can, for instance, make use of a previous scan of the same body, such as a low intensity scout scan, or from other already available data known about the body.

By having separate emission regions 31, 32 each illuminating substantially adjacent parts of the surface of the detector 2 even further advantages are possible compared to known stereo tube imaging wherein both beams overlap each other across at least most of the surface of the detector 2.

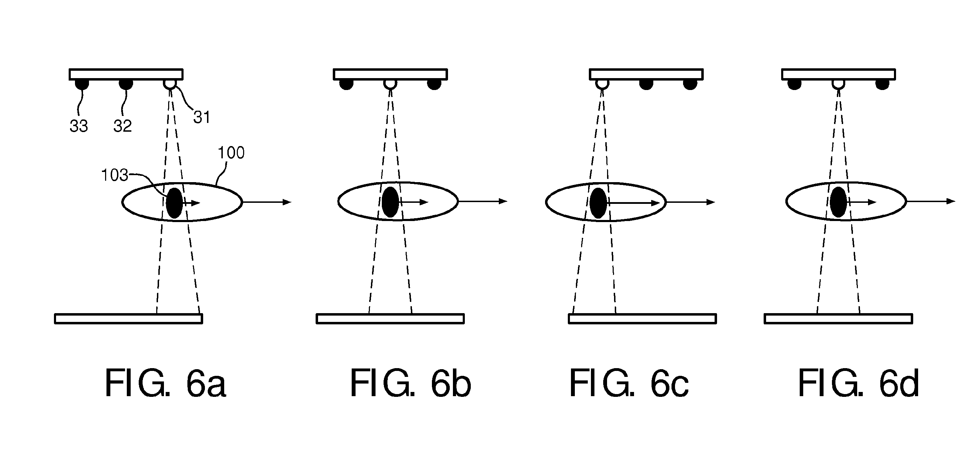

Figures 6a to 6d show an embodiment wherein a third emission region 33 is present in radiation unit 3, which allows for compensating an even further change in object speed v. The travelling object of interest 103 (e.g. a contrast agent bolus travelling through a blood vessel in a body 100) may be imaged by any of the emission regions 31, 32 or 33 individually, sequentially or together. This configuration may require more technical and computational complexity, but allows for even more flexibility in tracking a bolus 103 travelling at non-constant speed through a body 100. In this embodiment, radiation beams 61, 62 and 63 each illuminate adjacent regions covering approximately one third of the surface of detector 2, reducing the received radiation per surface area even further. This embodiment may be even further expanded using four or more emission regions, but space on the gantry 4 of the imaging system 1 is limited and driving mechanics and computation will become more complex, making it practically more challenging.

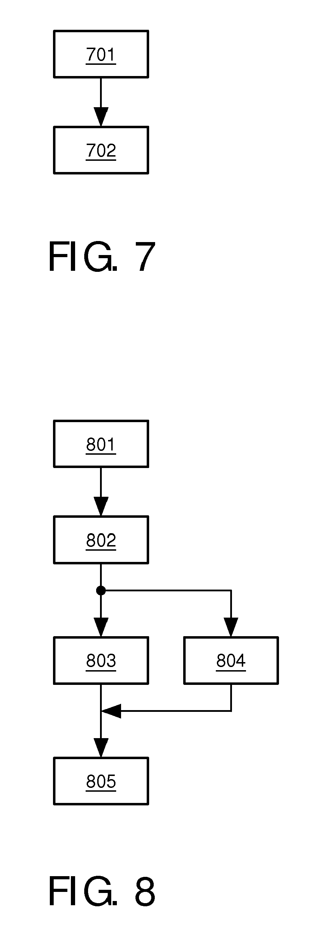

Figure 7 shows a flowchart of a method to scan an object of interest with an imaging system according to the present invention. First the object of interest is irradiated 701 from the first radiation emission region and then the object of interest is irradiated 702 from the second radiation emission region.

Figure 8 shows a flowchart of a method to track an object of interest travelling through a body with an imaging system according to the present invention. After the body has been placed in the examination region of the imaging system, radiation is emitted 801 from the first radiation emission region. From the moment the object of interest arrives in the examining region, the body is moved 802 through the examination region at the same speed and in the same direction as the object of interest. The object of interest location is determined 803 and it is determined whether the object of interest location is within the examination region. Radiation from the second radiation emission region is emitted 805 when the determined object of interest location is outside the examination region. As an optional step, the location of the object of interest relative to the examination region for at least part of a remaining length of the body through which the object of interest will travel is predicted 805 and radiation is emitted 805 from the second radiation emission region when the immediately upcoming object of interest location is predicted to be outside the examination region.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage.

## Claims

1. Imaging system comprising a detection unit comprising a detector surface and a radiation unit comprising a first radiation emission region configured to emit radiation towards a first area of the detector surface area, and a second radiation emission region configured to emit radiation towards a second area of the detector surface area, **characterized in that** the first area of the detector surface is different from the second area of the detector.

2. Imaging system according to claim 1, wherein in the first area of the detector surface and the second area of the detector surface do not overlap substantially.

3. Imaging system according to any of the claims 1 to 2, wherein the first area of the detector surface and the second area of the detector surface together cover substantially the entire detector surface.

4. Imaging system according to any of the claims 1 to 3, wherein the radiation unit is configured to switch between emitting radiation from only the first radiation emission region, only the second radiation emission region or from both simultaneously.

5. Imaging system according to any of the claims 1 to 4, wherein the radiation unit is configured such that the intensity of radiation emitted from the first radiation emission region may be gradually decreased and the intensity of radiation emitted from the second radiation emission region may be gradually increased.

6. Imaging system according to any of the claims 1 to 5, further comprising a first collimator for variably collimating radiation from the first emission region and a second collimator for variably collimating radiation from the second emission region.

7. Imaging system according to any of the claims 1 to 6, wherein the detector is contiguous.

8. Imaging system according to any of the claims 1 to 7 being an x-ray imaging system or a computed tomography imaging system.

9. Method to scan an object of interest with an imaging system, said imaging system comprising a detector unit with a detector surface and a radiation unit comprising a first radiation emission region configured to emit radiation towards a first area of the detector surface, and a second radiation emission region configured to emit radiation towards a second area of the detector surface, the method comprising the steps of:
- irradiating the object of interest from the first radiation emission region, and
- irradiating the object of interest from the second radiation emission region.

10. Method according to claim 9, wherein in the first area of the detector surface and the second area of the detector surface do not overlap, preferably wherein the first area of the detector surface and the second area of the detector surface are adjacent to each other.

11. Method according to claim 9 or 10, wherein the object of interest is first irradiated from only the first radiation emission region, followed by irradiating the object of interest from only the first radiation emission region.

12. Method according to claim 9, 10 or 11, wherein the object of interest travels through a body at an object speed and in an object direction, comprising:
- placing the body in an examination region of the imaging system;
- emitting radiation from the first radiation emission region; and
- from the moment the object of interest arrives in the examining region, moving the body through the examination region at the object speed and in the object direction; **characterized in that**
- the imaging system further comprises an object of interest locator unit; and the method further comprises the steps of:
- determining an object of interest location with the object of interest locator;
- determining whether the object of interest location is within the examination region;
- emitting radiation from the second radiation emission region when the determined object of interest location is outside the examination region.

13. Method according to claim 12, wherein the second radiation emission region is behind the first emission region with respect to the object direction and emitting radiation from the second emission region when the examination region outruns the object of interest.

14. Method according to claim 12, wherein the first radiation emission region is behind the second emission region with respect to the object direction and emitting radiation from the second emission region when the object of interest outruns the examination region.

15. Method according to any of the claims 10 to 14, wherein the detection unit comprises an object of interest location prediction unit for predicting a location of the object of interest relative to the examination region for at least part of a remaining length of the body through which the object of interest will travel and wherein radiation is emitted from the second radiation emission region when the immediately upcoming object of interest location is predicted to be outside the examination region.

16. Method according to any of the claims 11 to 15, wherein the body is a blood vessel and the object of interest is a contrast agent bolus.
